(19) Europäisches Patentamt
European Patent Office
Office européen des brevets

(11) Veröffentlichungsnummer: **0 184 711**
**A2**

# (12) EUROPÄISCHE PATENTANMELDUNG

(21) Anmeldenummer: **85114958.3**

(22) Anmeldetag: **26.11.85**

(51) Int. Cl.⁴: **C 09 B 19/02**
// C07D498/22

(30) Priorität: **08.12.84 DE 3444886**

(43) Veröffentlichungstag der Anmeldung: **18.06.86**
**Patentblatt 86/25**

(84) Benannte Vertragsstaaten: **CH DE FR GB LI**

(71) Anmelder: **BAYER AG, Konzernverwaltung RP**
**Patentabteilung, D-5090 Leverkusen 1 Bayerwerk (DE)**

(72) Erfinder: **Franke, Günter, Dr.,**
**Landrat-Trimborn-Strasse 60, D-5653 Leichlingen 1 (DE)**
Erfinder: **Harms, Wolfgang, Dr., Walter-Flex-Strasse 21,**
**D-5090 Leverkusen (DE)**

## (54) Dioxazinpigmente.

(57) Die neuen Dioxazinpigmente der allgemeinen Formel

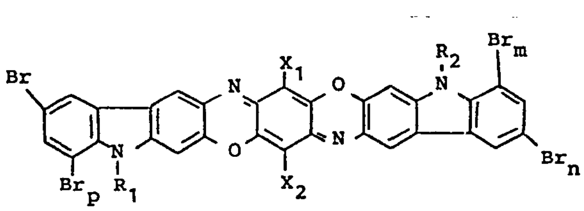

in der

$X_1$, $X_2$ Wasserstoff, Fluor, Chlor, Nitro, -R',

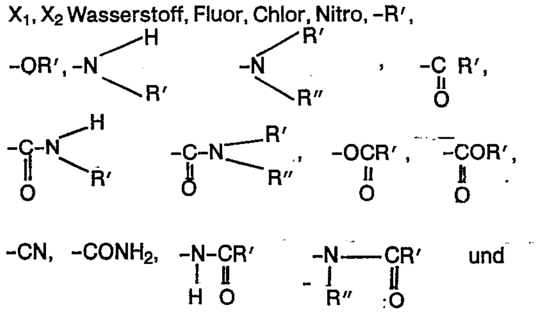

$R^1$, $R^2$ Wasserstoff, $-R'_1$, $-R'_1-O-R''_1$, $-R_1'\underset{O}{\underset{\|}{C}}-$ und

$R'_1$ $-SO_2-$ bedeuten, wobei
R', R'', $R'_1$, $R''_1$ für Alkyl, Cycloalkyl, Aryl und Aralkyl stehen und
m, n, p 0 oder 1 bezeichnen,
eignen sich zur Herstellung von sehr echt pigmentierten Systemen.

EP 0 184 711 A2

BAYER AKTIENGESELLSCHAFT 5090 Leverkusen, Bayerwerk
Konzernverwaltung RP
Patentabteilung PG/ABc

## Dioxazinpigmente

Die Erfindung betrifft Dioxazinpigmente der allgemeinen Formel

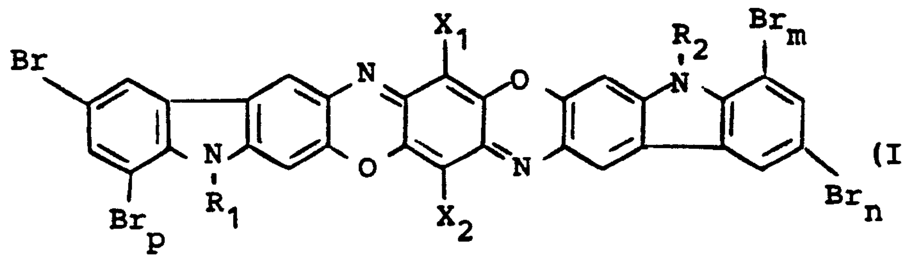

(I)

in der

$X_1$, $X_2$ Wasserstoff, Fluor, Chlor, Nitro, -R',

-OR', 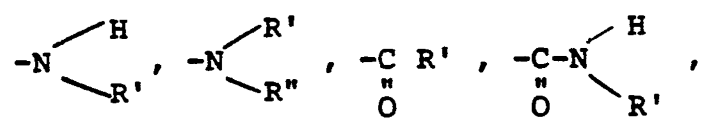

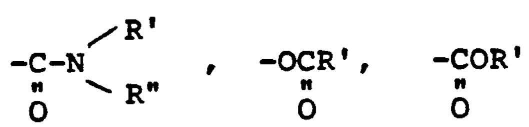 -CN, $-CONH_2$,

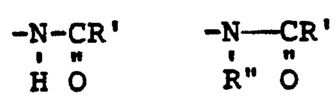 und

$R^1$, $R^2$ Wasserstoff, $-R_1'$, $-R_1'-O-R_1''$, $-R_1'\underset{\underset{O}{\|}}{C}-$ und $R_1'-SO_2-$ bedeuten, wobei

$R'$, $R''$, $R_1'$, $R_1''$ für Alkyl, Cycloalkyl, Aryl und Aralkyl stehen und

m, n, p 0 oder 1 bezeichnen.

Alkyl steht bevorzugt für $C_1$-$C_4$-Alkyl.

Cycloalkyl steht bevorzugt für Cyclopentyl und Cyclohexyl.

Aryl steht bevorzugt für Phenyl oder p-Tolyl. Aralkyl steht bevorzugt für Benzyl und Phenylethyl.

Bevorzugt sind Pigmente der Formel

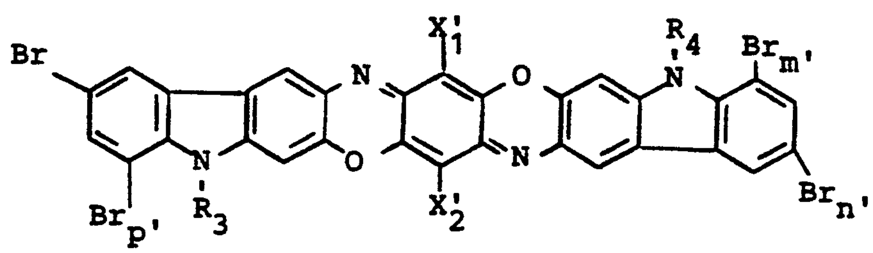

(II)

in der

$X_1'$, $X_2'$ Wasserstoff und Chlor,

$R_3$, $R_4$ Wasserstoff, $C_1$-$C_4$-Alkyl, $C_1$-$C_4$-Alkylcarbonyl, $C_1$-$C_4$-Alkylsulfonyl, Benzoyl, Phenylsulfonyl,

p-Tolylsulfonyl bedeuten und

m', n', p' 0 oder 1 bezeichnen, wobei die Summe aus m' + n' + p' 0 bis 2 beträgt.

Bei bevorzugten Pigmenten der Formeln (I) und (II) sind für den Fall, daß n oder n' = 0 sind auch m und m' = 0.

Bei besonders bevorzugten Pigmenten der Formel (II) sind $X_1'$ und $X_2'$ und/oder $R_3$ und $R_4$ gleich.

Ganz besonders wichtig sind darüber hinaus die Farbstoffe der Formel (II), bei denen $R_3$, $R_4$ $C_1$-$C_4$-Alkyl, insbesondere Ethyl, und m = p = 0 bezeichnen.

Die Herstellung der Pigmente der Formel (I) erfolgt beispielsweise dadurch, daß man gemäß Schema 1 Aminocarbazole der Formeln (IIIa + IIIb) mit Chinonen der Formel (IV) zu Zwischenprodukten der Formel (V) kondensiert und diese nachfolgend durch doppelten Ringschluß in die erfindungsgemäßen Farbstoffe überführt.

Schema 1

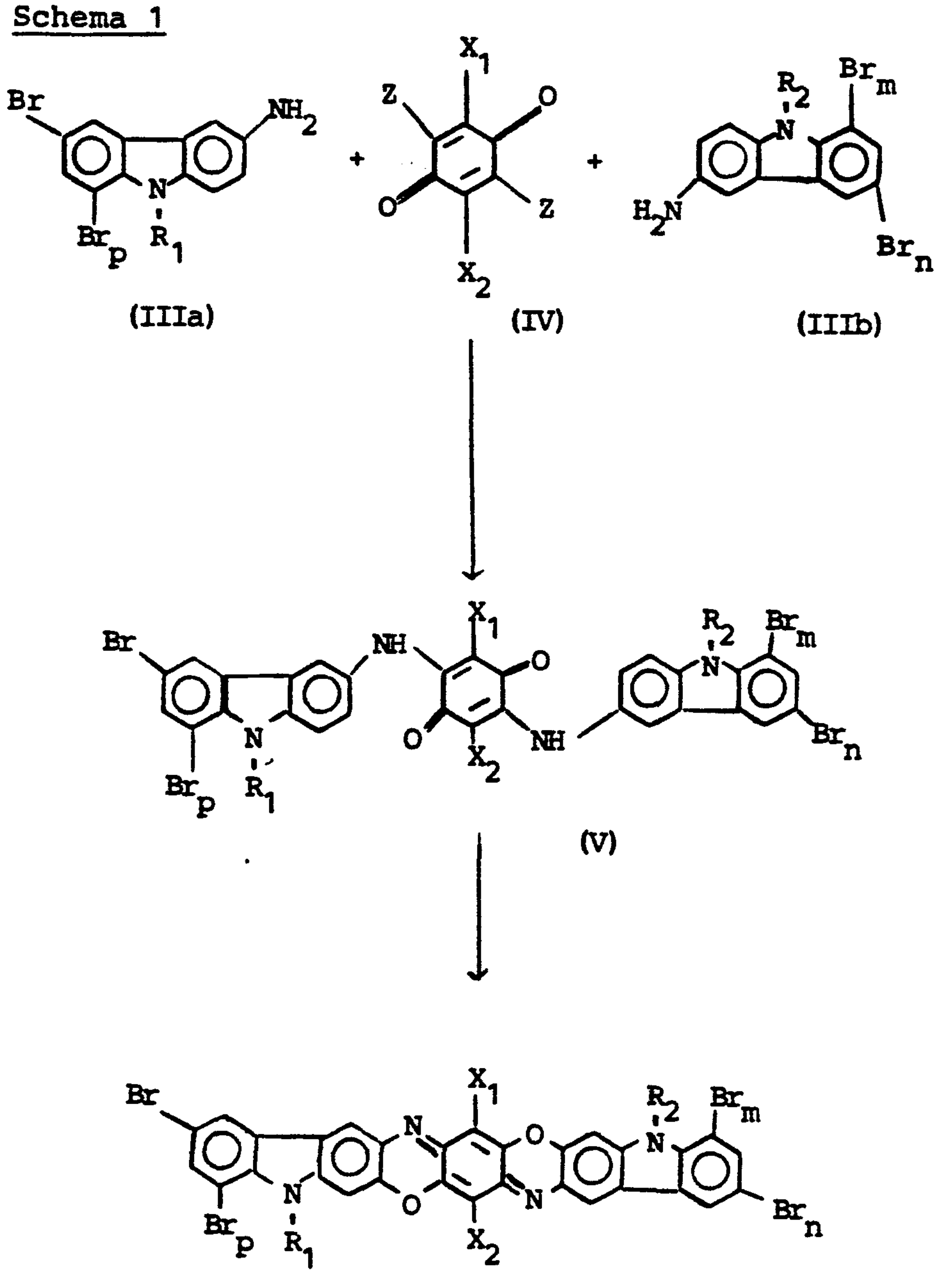

(IIIa) (IV) (IIIb)

(V)

(I)

In den Formeln IIIa, IIIb, IV und V haben $X_1$, $X_2$, $R_1$, $R_2$, m, n und p die zu Formel I angegebenen Bedeutungen. Z bezeichnet vorzugsweise Halogen, insbesondere Chlor, Hydroxy, Alkoxy und Aryloxy, insbesondere Phenoxy.

Als Mittelkomponenten der Formel (IV) kommen z.B. Verbindungen der Formeln (VI) und (VII) in Frage

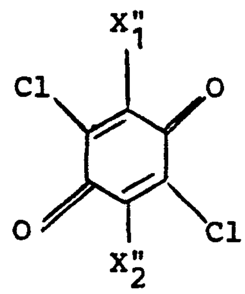

(VI)

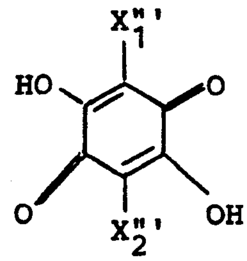

(VII)

worin $X_1''$ und $X_2''$ dasselbe wie $X_1$ und $X_2$ jedoch kein CN bzw. $X_1'''$ und $X_2'''$ dasselbe wie $X_1$ und $X_2$ jedoch weder Halogen noch CN bedeuten. Ist gewünscht, daß in den Pigmenten der Formel (I) $X_1$ und $X_2$ CN bedeutet, so erfolgt die Herstellung dieser Farbstoffe derart, daß man in Verbindungen der Formel (I) mit $X_1 = X_2 = Cl$ die Chloratome gegen CN austauscht, z.B. durch Umsetzung mit Kupfercyanid.

Wird die Synthese von I gemäß Schema 1 mit einer Mittelkomponente der Formel (VI) durchgeführt, so ist es von Vorteil, dies in einer Eintopfreaktion, also ohne Zwischenisolierung von (V), zu tun.

Die Verbindungen der Formel (I) oder ihre Gemische eignen sich, vorzugsweise nach üblicher Trocken- oder Naßkonditionierung aufgrund ihrer guten Pigmenteigenschaften für die verschiedensten Pigmentapplikationen. So

können sie zur Herstellung von sehr echt pigmentierten Systemen, wie Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischem Weißpigmenten wie Titandioxid (Rutil) verstanden werden. Zubereitungen sind z.B. Flushpasten mit organischen Flüssigkeiten und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen. Insbesondere eignen sich die erfindungsgemäßen Pigmente für Anstrichmittel zum Einsatz in Automobillacken, namentlich für Metalliclackierungen und zum Färben von makromolekularen Stoffen. Diese können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylestern oder Methacrylestern, Acrylnitirl, Acrylamid, Butadien, Styrol sowie Polyurethane und Polycarbonate. Die mit dem beanspruchten Produkt pigmentierten Stoffe können in beliebiger Form vorliegen.

0184711

Die erfindungsgemäßen Pigmente sind ausgezeichnet lösungsmittel-, öl- und wasserecht, säure-, alkali- und kalkecht, überlackier-, überspritz-, migrations- und sublimierecht, hitze- und vulkanisierbeständig und insbesondere licht- und wetterecht.

Neben ihrer Verwendung als Pigmente können die Verbindungen der Formel (I) nach üblichen Methoden sulfoniert werden und so als Direktfarbstoff Verwendung finden.

Die Verbindungen der Formel (I) zeichnen sich gegenüber ihren bromfreien Analogen durch eine deutlich rötere Nuance aus und zeigen ihnen gegenüber deutliche Vorteile z.B. in den Überlackier- und Überspritzechtheiten.

Beispiele

Beispiel 1

a) 9-Ethyl-3-nitrocarbazol wird entsprechend D.R.P. Org. Chem. 6, 2407 in Eisessig bromiert und nachfolgend in üblicher Weise mit Natriumsulfid in Ethanol zu

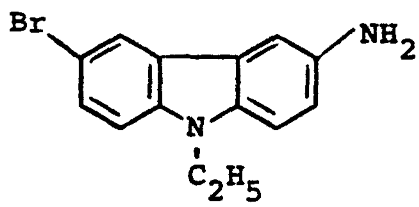

reduziert.

b) 99,5 g der vorstehenden Verbindung und 23,9 g 2,5-Dihydroxy-p-benzochinon werden in einer Mischung von 685 ml Eisessig und 33 ml 10 proz. Schwefelsäure 1 Stunde unter Rückfluß erhitzt. Nach Kaltrühren wird abgesaugt, mit 1,5 l Eisessig, dann mit 4 l heißem Wasser gewaschen und bei 100°C getrocknet. Man erhält 104 g der Verbindung

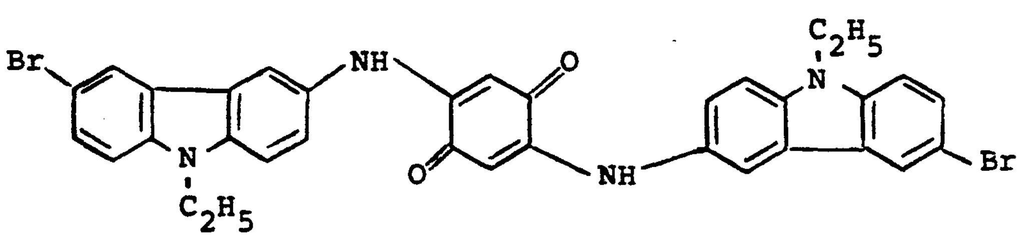

c) 26,0 g vorstehender Verbindung und 8,1 g Benzolsulfonsäurechlorid werden in 120 ml Nitrobenzol erhitzt und 1 Stunde unter Rückfluß gehalten. Nach Abkühlen auf 60-70°C wird mit 500 ml Methanol verdünnt und durch die Mischung Ammoniakgas geleitet. Man saugt ab, wäscht zunächst mit Methanol, dann mit Nitrobenzol und schließlich wieder mit Methanol. Trocknen bei 100°C ergibt 19,7 g des nachstehenden Dioxazins

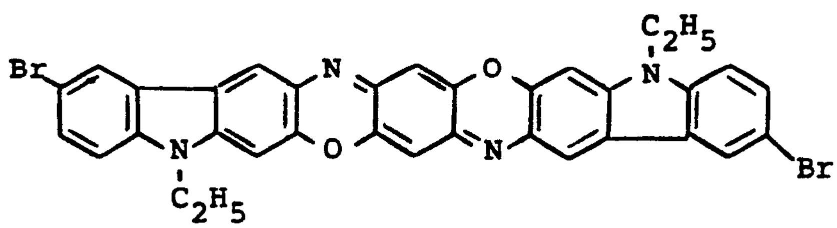

mit einem Bromgehalt von 23,0 % (berechnet 23,56 %).

Beispiel 2

43,2 g 3-Amino-6-brom-9-ethylcarbazol (Beispiel 1a) und 22,2 g Chloranil werden in 320 ml o-Dichlorbenzol verrührt. Unter Kühlung werden 15,9 g Triethylamin zugegeben und es wird 2 Stunden bei 30-35°C gerührt. Nach Zusatz von 19,2 g Toluolsulfonsäurechlorid wird auf 170-180°C erhitzt und 2 h bei dieser Temperatur gerührt. Man saugt heiß ab, wäscht mit Nitrobenzol, dann mit Methanol und zuletzt mit Wasser und trocknet bei 100°C. Man erhält 48,3 g des folgenden Dioxazins

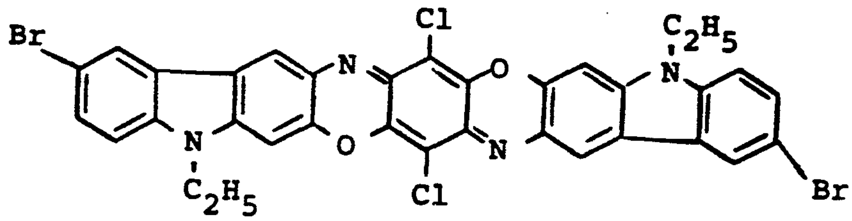

mit einem Gehalt von 21,5 % Brom und 9,4 % Chlor (berechnet 21,39 % bzw. 9,49 %).

Beispiel 3

Werden anstelle der 43,2 g des 3-Amino-6-brom-9-ethylcarbazols äquimolare Mengen der analog gewonnenen Verbindungen

a) 3-Amino-6-brom-9-methylcarbazol

b) 3-Amino-6-brom-9-butylcarbazol

eingesetzt und im übrigen wie in Beispiel 2 verfahren, erhält man in beiden Fällen Pigmente, die in ihren färberischen Eigenschaften dem gemäß Beispiel 2 erhaltenen entsprechen.

Beispiel 4

Wird anstelle der 43,2 g 3-Amino-6-brom-9-ethylcarbazol in Beispiel 2 eine Mischung von 21,6 g dieser Verbindung

mit 15,8 g 3-Amino-9-ethylcarbazol eingesetzt und im Übrigen wie in Beispiel 2 verfahren, erhält man 41,2 g des Dioxazins

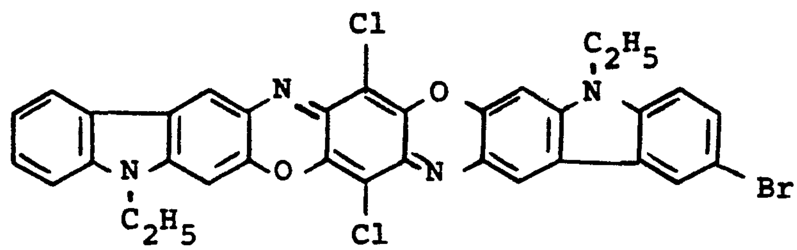

mit einem Gehalt von 12,3 % Brom und 10,8 % Chlor (berechnet 11,95 % bzw. 10,61 %).

Beispiel 5

a) Bromiert man 9-Ethyl-3-nitrocarbazol analog zu D.R.P. Org. Chem. 6, 2407 in Eisessig, setzt dabei jedoch das Zweifache der dort angegebenen Menge Brom ein, erhält man 6,8-Dibrom-9-ethyl-3-nitrocarbazol, das in üblicher Weie mit Natriumsulfid in Ethanol zu

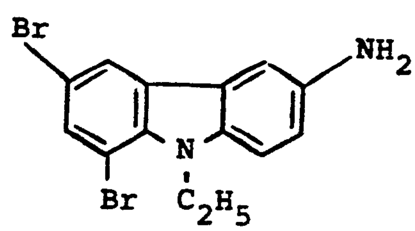

reduziert wird.

b) 131,8 g der vorgenannten Verbindung und 24,9 g 2,5-Dihydroxy-p-benzochinon werden in einer Mischung aus 710 ml Eisessig und 34 ml 10 proz. Schwefelsäure 2 Stunden unter Rückfluß gekocht. Nach Erkalten wird abgesaugt, zunächst mit Eisessig, dann mit Wasser gewaschen, und bei 100°C getrocknet. Man erhält 138,5 g der nachfolgenden Verbindung

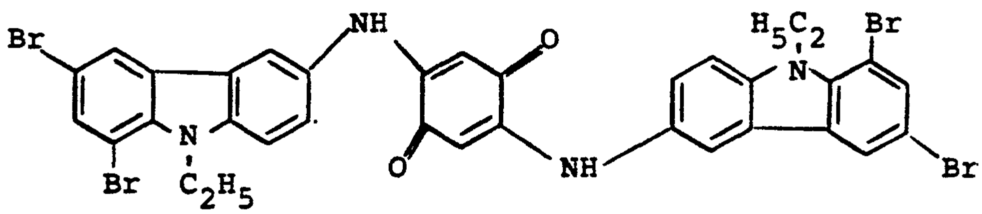

c) 84,0 g des vorgenannten Zwischenprodukts und 21,0 g Toluolsulfonsäure werden in 2,5 l Nitrobenzol erhitzt und 2 Stunden unter Rückfluß gehalten. Man saugt heiß ab, wäscht mit Nitrobenzol, dann mit Methanol und trocknet bei 80°C. Man erhält 66,7 g eines Dioxazins der Formel

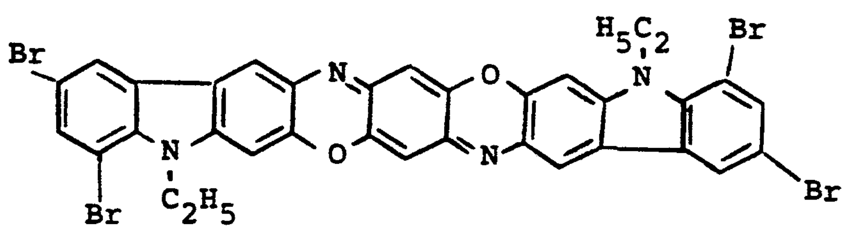

mit einem Gehalt an Brom von 37,7 % (berechnet 38,22 %).

Beispiel 6

Es werden 55,2 g 3-Amino-6,8-dibrom-9-ethylcarbazol (Beispiel 5a) und 22,2 g Chloranil in 500 ml o-Dichlorbenzol verrührt. Wird nachfolgend genau wie in Beispiel 2 verfahren, erhält man 51,5 g des folgenden Dioxazins

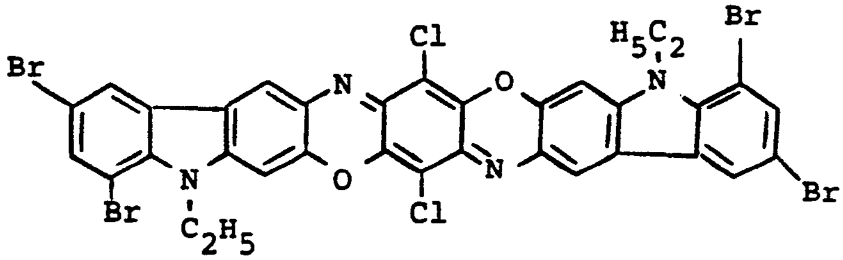

mit einem Gehalt von 32,4 % Brom und 8,1 % Chlor (berechnet 32,31 % bzw. 8,19 %).

Beispiel 7

Wird anstelle der 43,2 g 3-Amino-6-brom-9-ethylcarbazol in Beispiel 2 eine Mischung von 27,6 g 3-Amino-6,8-dibrom-9-ethylcarbazol und 15,8 g 3-Amino-9-ethylcarbazol eingesetzt und ansonsten so wie in Beispiel 2 verfahren, erhält man 46,9 g des Dioxazins

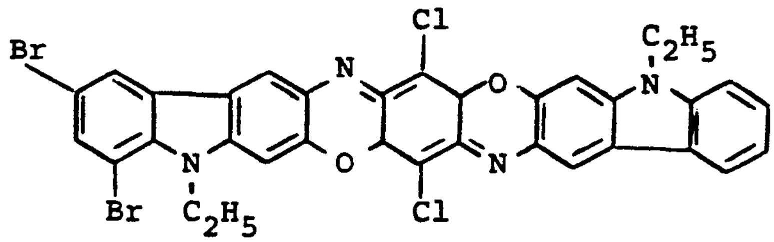

mit einem Gehalt von 21,7 % Brom und 9,6 % Chlor (berechnet 21,39 % bzw. 9,49 %).

## Patentansprüche

1. Dioxazinpigmente der allgemeinen Formel

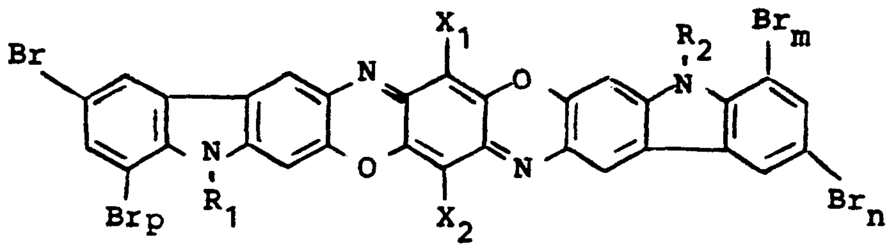

(I)

in der

$X_1$, $X_2$ Wasserstoff, Fluor, Chlor, Nitro, -R',

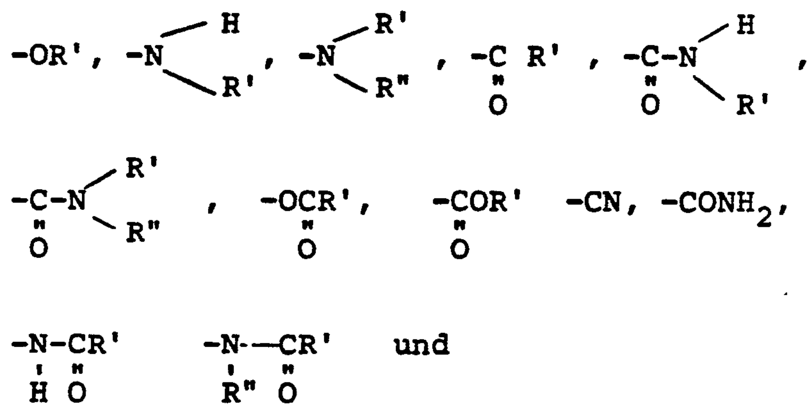

und

$R^1$, $R^2$ Wasserstoff, $-R_1'$, $-R_1'-O-R_1''$, $-R_1'\underset{\overset{\|}{O}}{C}-$ und $R_1'$ $-SO_2-$ bedeuten, wobei

R', R", $R_1'$, $R_1''$ für Alkyl, Cycloalkyl, Aryl und Aralkyl stehen und

m, n, p   0 oder 1 bezeichnen.

2. Dioxazinpigmente gemäß Anspruch 1, bei denen $R'$, $R''$, $R_1'$, $R_1''$ für $C_1$-$C_4$-Alkyl, Cyclopentyl, Cyclohexyl, Phenyl, p-Tolyl, Benzyl und Phenylethyl stehen.

3. Dioxazinpigmente der Formel

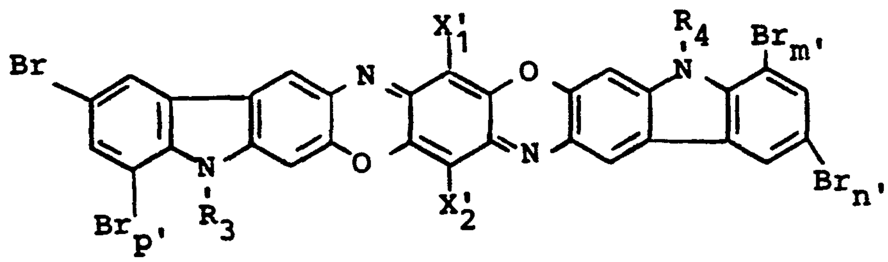

(II)

in der

$X_1'$, $X_2'$   Wasserstoff und Chlor,

$R_3$, $R_4$   Wasserstoff, $C_1$-$C_4$-Alkyl, $C_1$-$C_4$-Alkylcarbonyl, $C_1$-$C_4$-Alkylsulfonyl, Benzoyl, Phenylsulfonyl, p-Tolylsulfonyl bedeuten und

m', n', p' 0 oder 1 bezeichnen, wobei die Summe aus m' + n' + p' 0, 1 oder 2 beträgt.

4. Dioxazinpigmente gemäß Anspruch 3, bei denen $X_1' = X_2'$.

5. Dioxazinpigmente gemäß den Ansprüchen 3 und 4, bei denen $R_3 = R_4$.

0184711

6. Dioxazinpigmente gemäß den Ansprüchen 3-5, bei denen $R_3$, $R_4$ $C_1$-$C_4$-Alkyl ist und m = p = 0 bezeichnen.

7. Dioxazinpigmente gemäß den Ansprüchen 1-6, bei denen für den Fall, daß n oder n' = O sind, auch m oder m' = 0 sind.

8. Dioxazinpigment der Formel

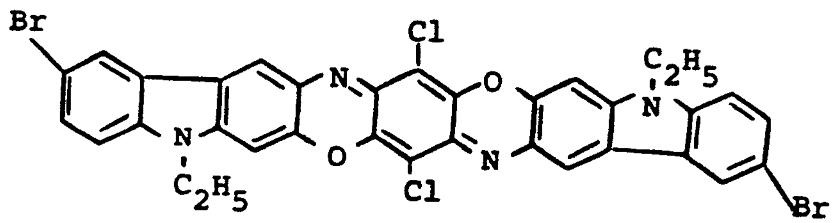

9. Dioxazinpigment der Formel

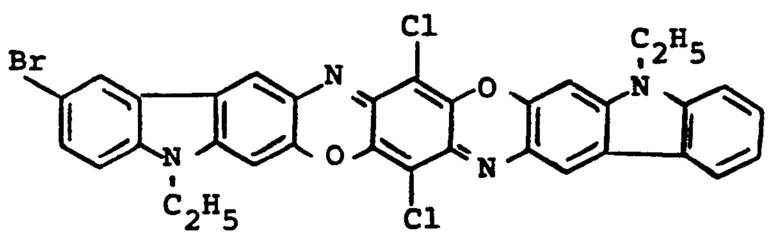

10. Verfahren zur Herstellung von pigmentierten Systemen, dadurch gekennzeichnet, daß man Dioxazinpigmente gemäß den Ansprüchen 1-9 verwendet.